(19) Europäisches Patentamt
European Patent Office
Office européen des brevets

(11) Numéro de publication : **0 560 683 A1**

# (12) DEMANDE DE BREVET EUROPEEN

(21) Numéro de dépôt : **93400625.5**

(22) Date de dépôt : **11.03.93**

(51) Int. Cl.[5] : **C07D 209/12,** A61K 7/13, C07D 409/10

(30) Priorité : **13.03.92 FR 9203049**

(43) Date de publication de la demande : **15.09.93 Bulletin 93/37**

(84) Etats contractants désignés : **AT BE CH DE DK ES FR GB GR IE IT LI NL PT SE**

(71) Demandeur : **L'OREAL
14, Rue Royale
F-75008 Paris (FR)**

(72) Inventeur : **Henrion, Jean-Christophe
8, rue Jolly
F-94160 Saint-Mandé (FR)**
Inventeur : **Philippe, Michel
3, rue de l'Aubépine
F-92160 Antony (FR)**
Inventeur : **Hocquaux, Michel
70, rue du Rendez-vous
F-75012 Paris (FR)**

(74) Mandataire : **Casalonga, Axel et al
BUREAU D.A. CASALONGA - JOSSE
Morassistrasse 8
D-80469 München (DE)**

## (54) Compositions de teinture des fibres kératiniques à base de monoindolyl 1,4-naphtoquinones ou leurs leucodérivés, nouveaux composés monoindolyl 1,4-naphtoquinones et leurs leucodérivés.

(57) Composition de teinture des fibres kératiniques, en particulier des cheveux humains, contenant dans un milieu approprié pour la teinture, au moins un composé répondant à l'une des deux formules suivantes (I) ou (II) :

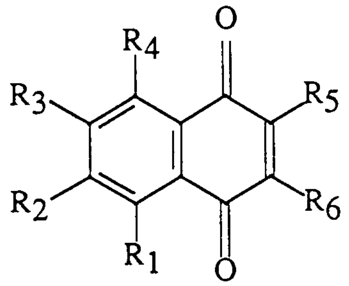

(I)

ou

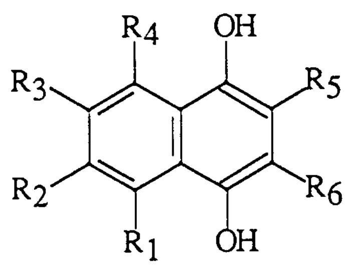

(II)

dans lesquelles :

$R_1$ à $R_4$, identiques ou différents, désignent hydrogène, OH, alcoxy ; deux au plus pouvant désigner OH ;

$R_5$ ou $R_6$ désignent hydrogène, OH, halogène, alkyle, alcoxy, phényle, thiophényle, adamantylméthyle, adamantylamino, alkylamino, pipéridyle, morpholinyle ou R de formule :

EP 0 560 683 A1

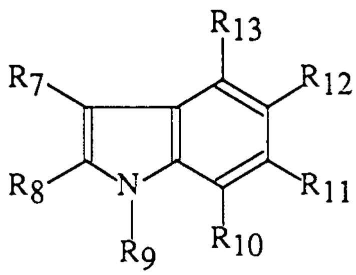

(III)

où

$R_7$ ou $R_8$ désignent hydrogène, alkyle, phényle ou une liaison covalente ; un des symboles $R_7$ et $R_8$ désigne une liaison covalente ;

$R_9$ désigne hydrogène ou alkyle ;

$R_{10}$ à $R_{13}$, identiques ou différents, désignent hydrogène, alkyle, halogène, alcoxy, alcoxycarbonyle, acyle, acyloxy, $NO_2$, OH, amino éventuellement monosubstitué, cyano, benzyloxy ;

$R_{11}$ et $R_{12}$ peuvent former méthylènedioxy ;

au plus un seul des radicaux peut désigner halogène, $NO_2$, alcoxycarbonyle, acyle, cyano, amino éventuellement monosubstitué ou acyloxy ;

au plus deux peuvent désigner OH, alcoxy, benzyloxy ;

un des radicaux $R_5$ et $R_8$ désigne un radical R.

La présente invention concerne de nouvelles compositions de teinture des fibres kératiniques à base de monoindolyl 1,4-naphtoquinones ou leurs leucodérivés, ainsi que les nouveaux composés monoindolyl 1,4-naphtoquinones et leurs leucodérivés.

On connaît dans l'état de la technique des produits d'addition de composés indoliques avec des quinones telles que la 1,4-naphtoquinone et ses dérivés.

L'article de PROTA (Tetrahedron Letters, Vol. 43, n° 12, pages 2749-54, 1987) décrit des produits d'addition du 5,6-dihydroxyindole, du 5,6-dihydroxy 1-méthylindole ou du 5,6-dihydroxy 2-méthylindole sur la 1,4-naphtoquinone.

L'article de BU'LOCK & MASON (J. Chem. Soc., pages 703 à 716, 1951) décrit les synthèses de la 2-(2-méthyl 3-indolyl)1,4-naphtoquinone et de la 2-(3-indolyl) 1,4-naphtoquinone.

L'article de PROTA (Gazetta Chemica, Italiana 119, 1989) décrit la synthèse de la 2-(3-méthyl 2-indolyl) 1,4-naphtoquinone.

L'article de PRASAD (Tetrahedron Letters, n° 15, pages 1361-1362, 1974) décrit la synthèse de la 3-(3-indolyl) 1,4-naphtoquinone.

Les demandes EP-0376776 et 0460996 décrivent un procédé de teinture en 2 étapes, mettant en oeuvre des mono- ou des dihydroxyindoles ou des aminoindoles qui sont oxydés, par des quinones, pour former un pigment mélanique, in situ dans les fibres kératiniques.

La demanderesse vient de découvrir de manière surprenante que les monoindolyl 1,4-naphtoquinones ou leurs leucodérivés de structure monoindolyl 1,4-naphtalènediol pouvaient être utilisés en teinture directe des fibres kératiniques et conduisaient, sans l'intermédiaire d'un système oxydant, à une palette variée de colorations.

La présente invention concerne donc de nouvelles compositions de coloration directe des fibres kératiniques à base de ces composés.

Un autre objet concerne les nouveaux composés présents dans ces compositions.

Un autre objet concerne les procédés de teinture directe des fibres kératiniques utilisant les compositions de l'invention.

Les compositions de teinture des fibres kératiniques, en particulier des cheveux humains, selon l'invention, sont caractérisées par le fait qu'elles contiennent dans un milieu approprié pour la teinture, au moins un composé répondant à l'une des formules suivantes :

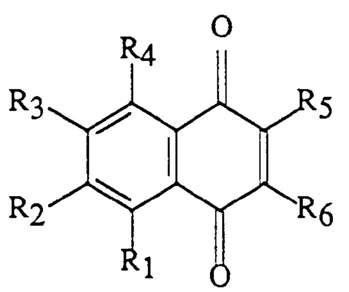

ou

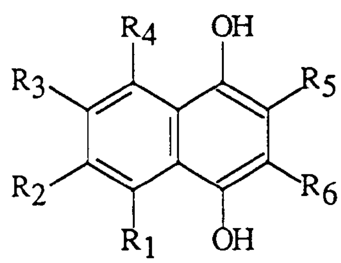

(I) (II)

dans lesquelles :

$R_1$ à $R_4$, identiques ou différents, désignent un atome d'hydrogène, un groupe OH, un radical alcoxy en $C_1$-$C_4$; deux au plus pouvant désigner OH;

$R_5$ ou $R_5$ désignent un atome d'hydrogène, OH, un halogène, un radical alkyle en $C_1$-$C_{12}$, alcoxy en $C_1$-$C_4$, phényle, thiophényle, adamantylméthyle, adamantylamino, alkyl($C_1$-$C_4$)amino, pipéridyle, morpholinyle ou un radical R de formule :

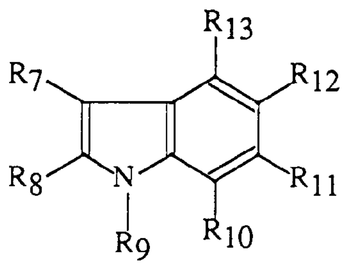

(III)

où

$R_7$ ou $R_8$ désignent un atome d'hydrogène, un radical alkyle en $C_1$-$C_4$, phényle ou une liaison covalente; un des symboles $R_7$ et $R_8$ désigne une liaison covalente;

$R_9$ désigne un atome d'hydrogène ou un radical alkyle en $C_1$-$C_4$;

$R_{10}$ à $R_{13}$, identiques ou différents, désignent un atome d'hydrogène, un radical alkyle en $C_1$-$C_4$, un halogène, un radical alcoxy en $C_1$-$C_4$, alcoxy($C_1$-$C_4$)carbonyle, acyle en $C_1$-$C_4$, acyl ($C_2$-$C_4$)oxy, $NO_2$, OH, amino éventuellement monosubstitué par un alkyle en $C_1$-$C_4$ ou un hydroxyalkyle en $C_1$-$C_4$, cyano ou benzyloxy;

au plus un seul des radicaux $R_{10}$ à $R_{13}$ peut désigner halogène, $NO_2$, alcoxycarbonyle, acyle, cyano, amino ou acyloxy;

au plus deux des radicaux $R_{10}$ à $R_{13}$ peuvent désigner OH, ou alcoxy ou benzyloxy;

$R_{11}$ et $R_{12}$ peuvent former un cycle méthylènedioxy;

un des radicaux $R_5$ et $R_6$ désigne un radical R.

Les composés préférentiels de la présente invention sont choisis parmi ceux de formules:

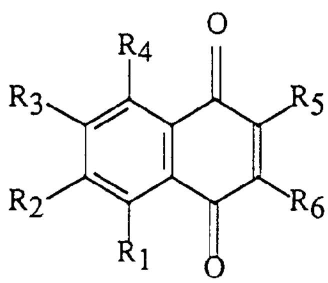

ou

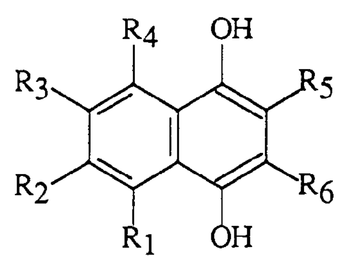

(I) (II)

dans lesquelles :

$R_1$ et $R_4$ désignent hydrogène, OH ou alcoxy;

$R_2$ et $R_3$ désignent hydrogène ou OH;

deux au plus des symboles $R_1$ à $R_4$ pouvant désigner OH;

$R_8$ ou $R_8$ désignent hydrogène, phényle, alkyle en $C_1$-$C_{12}$, adamantylméthyle, OH, méthoxy, chlore, brome, pipéridyle, thiophényle ou le radical R de formule :

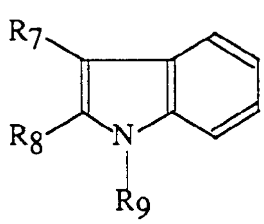

(III')

où

$R_9$ désigne hydrogène ou méthyle;

$R_7$ ou $R_8$ désignent hydrogène, méthyle, phényle ou une liaison covalente;

l'un des symboles $R_7$ et $R_8$ désigne une liaison covalente et l'un des radicaux $R_5$ et $R_8$ désigne un groupe R.

Les composés particulièrement préférés sont choisis parmi :

- la 2-(1-méthyl 3-indolyl)1,4-naphtoquinone,
- la 2-(2-méthyl 3-indolyl)1,4-naphtoquinone,
- la 2-(2-phényl 3-indolyl)1,4-naphtoquinone,
- la 2-(1,2-diméthyl 3-indolyl)1,4-naphtoquinone,
- la 3-(1-méthyl 3-indolyl)5-hydroxy 1,4-naphtoquinone,
- la 2-(2-méthyl 3-indolyl)5-hydroxy 1,4-naphtoquinone,
- la 3-(2-phényl 3-indolyl)5-hydroxy 1,4-naphtoquinone,
- la 2-(2-méthyl 3-indolyl)3-phényl 1,4-naphtoquinone,
- la 2-(2-phényl 3-indolyl)3-phényl 1,4-naphtoquinone,
- la 2-(1-méthyl 3-indolyl)3-phényl 1,4-naphtoquinone,
- la 2-(2-méthyl 3-indolyl)3-(1-pentyl)1,4-naphtoquinone,
- la 3-undécyl 2-(1,2-diméthyl 3-indolyl)1,4-naphtoquinone,
- la 3-undécyl 2-(2-phényl 3-indolyl)1,4-naphtoquinone,
- la 2-(2-méthyl 3-indolyl)3-(1-adamantyl)méthyl 1,4-naphtoquinone,
- la 2-(2-méthyl 3-indolyl)3-hydroxy 1,4-naphtoquinone,
- la 2-(2-phényl 3-indolyl)3-méthoxy 1,4-naphtoquinone,
- la 2-(1-méthyl 3-indolyl)3-chloro 1,4-naphtoquinone,
- la 3-(2-méthyl 3-indolyl)2-bromo 1,4-naphtoquinone,
- la 2-(2-méthyl 3-indolyl)3-(1-pipéridyl)1,4-naphtoquinone,
- la 2-(1,2-diméthyl 3-indolyl)3-(1-pipéridyl)1,4-naphtoquinone,
- la 2-(1-méthyl 3-indolyl)3-thiophényl 1,4-naphtoquinone,
- la 2-(2-méthyl 3-indolyl)3-thiophényl 1,4-naphtoquinone,
- la 2-(3-indolyl)5,8-dihydroxy 1,4-naphtoquinone,
- la 2-(3-méthyl 2-indolyl) 1,4-naphtoquinone,

ainsi que leurs dérivés de réduction correspondants de structure 1,4-dihydroxynaphtalène.

Certains des composés de formule (I) telle que définie ci-dessus sont connus et ont été décrits dans les documents cités plus haut.

Les composés nouveaux conformes à la présente invention, sont choisis parmi ceux de formule (I) ou (II) telles que définies précédemment, pour lesquels les radicaux $R_1$ à $R_{13}$ et R, ont les mêmes indications définies ci-dessus, sous réserve que :

a) $R_{11}$ et $R_{12}$ ne peuvent désigner simultanément OH lorsque $R_1$ à $R_4$, $R_6$ et $R_{10}$ et $R_{13}$ désignent hydrogène et $R_8$ et $R_9$ désignent tous deux hydrogène ou bien lorsque l'un des deux radicaux $R_8$ et $R_9$ désigne $CH_3$ et l'autre hydrogène;

b) $R_8$ ne peut désigner méthyle, lorsque $R_1$ à $R_4$, $R_6$, $R_9$ et $R_{10}$ à $R_{13}$ désignent hydrogène et $R_7$ désigne une liaison covalente;

c) $R_7$ ne peut désigner méthyle lorsque $R_1$ à $R_4$, $R_6$, $R_9$ et $R_{10}$ à $R_{13}$ désignent hydrogène et $R_8$ désigne une liaison covalente;

d) au moins l'un des radicaux $R_1$ à $R_4$, $R_8$ ou $R_8$ et $R_8$ à $R_{13}$ est différent d'hydrogène lorsque $R_7$ désigne une liaison covalente.

Les composés de formule (I) conformes à la présente invention peuvent être obtenus par condensation en catalyse acide d'un composé indolique de formule suivante :

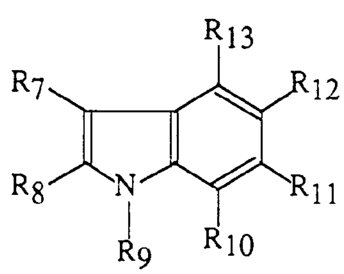

(1)

dans laquelle :

$R_7$ ou $R_8$ désigne un atome d'hydrogène, un radical alkyle en $C_1$-$C_4$, phényle;

l'un de ces radicaux désignant l'hydrogène;

$R_9$ à $R_{13}$ ont les mêmes significations indiquées ci-dessus dans la formule (III), sur une 1,4-naphtoquinone de formule :

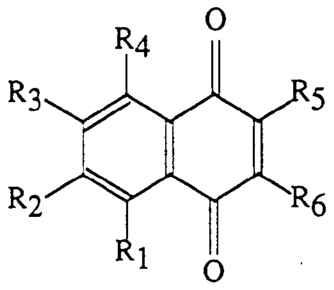

(2)

dans laquelle :

$R_1$ à $R_4$ ont les mêmes significations indiquées dans la formule (I) précédente;

$R_5$ ou $R_6$ désignent un atome d'hydrogène, OH, halogène, un alkyle en $C_1$-$C_{12}$, alcoxy en $C_1$-$C_4$, phényle, thiophényle, adamantylméthyle, adamantylamino, alkylamino en $C_1$-$C_4$, pipéridyle, morpholinyle;

l'un au moins des radicaux $R_5$ ou $R_6$ désignant l'hydrogène.

Le milieu réactionnel utilisé est de préférence constitué par de l'acide acétique glacial, l'acide acétique aqueux, l'éthanol avec quelques gouttes d'acide chlorhydrique, selon BU'LOCK & MASON (J. Chem. Soc. 1951, 703). On opère généralement à température ambiante ou au reflux de l'acide acétique.

On peut également effectuer cette condensation dans du chloroforme en présence d'acide tel que l'acide paratoluènesulfonique à température ambiante ou au reflux, ou bien dans le chloroforme seul au reflux.

Les composés de formule (I) telle que définie précédemment où les radicaux $R_5$ et $R_5$ sont différents de l'hydrogène, peuvent également être obtenus par substitution d'une 1,4-naphtoquinone de formule (2) - telle que définie ci-dessus où au moins un des radicaux $R_5$ et $R_6$ désigne un groupe nucléophile tel que OH, Br, Cl, méthoxy, thiophényle ou pipéridyle - par un composé indolique de formule (1) telle que définie précédemment.

On opère de manière préférentielle dans un milieu réactionnel constitué d'éthanol absolu au reflux.

Les composés de formule (II) de l'invention, de structure monoindolyl 1,4-dihydroxynaphtalène, sont obtenus à partir des composés monoindolyl 1,4-naphtoquinone de formule (I), par réduction avec le dithionite de sodium.

L'indolylnaphtoquinone est mise en solution dans l'éther éthylique sous argon, puis agitée en présence de deux équivalents de dithionite de sodium en solution aqueuse à température ambiante, jusqu'à décoloration totale (12 heures). On décante, sèche la phase organique et évapore le solvant.

Les compositions de teinture conformes à la présente invention comprennent de préférence un milieu approprié pour la teinture constitué par de l'eau, un solvant ou un mélange d'eau et d'un ou plusieurs solvant(s) cosmétiquement acceptable(s).

Les solvants sont choisis plus particulièrement parmi les alcools inférieurs en $C_1$-$C_6$, les alkylèneglycols comme l'éthylèneglycol ou le propylèneglycol, les éthers de glycol comme les éthers monométhylique, monoéthylique ou monobutylique de l'éthylèneglycol, l'acétate de monoéthyléther de l'éthylèneglycol, les monométhyléthers du propylèneglycol et du dipropylèneglycol, le lactate de méthyle.

Les solvants particulièrement préférés sont l'alcool éthylique et le propylèneglycol.

Les colorants de formule (I) ou (II) sont présents dans des concentrations comprises de préférence entre 0,01 et 10% en poids et en particulier entre 0,05 et 5% en poids par rapport au poids total de la composition.

Les compositions conformes à l'invention peuvent également contenir d'autres colorants directs différents de ceux des formules (I) et (II), tels que les dérivés nitrés de la série benzénique, des azoïques, des anthraquinoniques et d'autres adjuvants habituellement utilisés en cosmétique tels que des agents tensio-actifs anioniques, cationiques, non-ioniques, amphotères ou leurs mélanges, des épaississants, des parfums, des séquestrants, des agents filmogènes, des agents de traitement des fibres kératiniques, des agents dispersants, des agents de conditionnement des fibres kératiniques, des conservateurs, des agents de gonflement des fibres kératiniques.

Les compositions selon l'invention peuvent se présenter sous diverses formes telles que des lotions plus ou moins épaissies ou gélifiées, des dispersions, des crèmes, des mousses, des émulsions. Elles peuvent notamment être conditionnées dans des dispositifs aérosols.

Un autre objet de l'invention concerne un procédé de teinture directe des fibres kératiniques, en particulier des cheveux humains, consistant à appliquer sur ces derniers une composition telle que définie précédemment, l'application étant éventuellement suivie d'un rinçage, les cheveux étant ensuite séchés. Dans le cas où l'application est suivie d'un rinçage, la composition est maintenue au contact des cheveux pendant 5 à 45 minutes et de préférence entre 10 et 30 minutes.

Les exemples qui suivent servent à illustrer l'invention sans toutefois présenter un caractère limitatif.

## EXEMPLES DE PREPARATION

### EXEMPLE 1

**2-(1-méthyl 3-indolyl)1,4-naphtoquinone** (1)

Dans un tricol de 100 ml purgé à l'argon, on agite 1 g (6,33 mmoles) de 1,4-naphtoquinone et 1,22 ml (9,50 mmoles) de 1-méthyl indole dans 20 ml d'acide acétique glacial à température ambiante et sous atmosphère inerte. On suit régulièrement l'évolution de la réaction en chromatographiant le mélange sur plaque de gel de silice. Au bout de 60 heures d'agitation, on ajoute 60 ml d'eau et lave à l'eau le précipité formé. Celui-ci est repris dans 100 ml de dichlorométhane, séché sur sulfate de magnésium calciné sec. On chasse le solvant sous vide et chromatographie le résidu noir sur colonne de gel de silice (Merck 40-63 μm) éluée par un mélange $CH_2Cl_2/C_6H_{12}$, pour obtenir 0,318 g (1,11 mmole) de 2-(1-méthyl 3-indolyl)1,4-naphtoquinone (1). PF. 171°C. Coloration bleue.

### EXEMPLE 2

**2-(2-phényl 3-indolyl)1,4-naphtoquinone** (2)

Selon les conditions de l'exemple 1, 15,8 g (100 mmoles) de 1,4-naphtoquinone réagissent avec 19,3g (100 mmoles) de 2-phénylindole pendant 1 heure pour donner 12,4 g (35,5 mmoles) de 2-(2-phényl 3-indolyl) 1,4-naphtoquinone (2). PF. 202-203 °C. Coloration bleue.

### EXEMPLE 3

**2-(1,2-diméthyl 3-indolyl)1,4-naphtoquinone** (3)

Selon les conditions de l'exemple 1, 5 g (31,6 mmoles) de 1,4-naphtoquinone réagissent avec 4,62 g (31,6 mmoles) de 1,2-diméthyl indole pendant 1 heure, pour donner 4,52 g (15 mmoles) de 2-(1,2-diméthyl 3-indolyl)1,4-naphtoquinone (3). PF. 187°C. Coloration bleue.

### EXEMPLE 4

**2-(2-méthyl 3-indolyl)5-hydroxy 1,4-naphtoquinone** (4)

Selon les conditions de l'exemple 1, 4,42 g de 5-hydroxy 1,4-naphtoquinone (25,4 mmoles) réagissent avec 2,22 g (16,9 mmoles) de 2-méthylindole pour conduire, après 24 heures d'agitation, à 1,40 g (4,62 mmoles) de 2-(2-méthyl 3-indolyl)5-hydroxy 1,4-naphtoquinone (4). PF. 97°-98°C. Coloration violette.

### EXEMPLE 5

**3-(2-phényl 3-indolyl)5-hydroxy 1,4-naphtoquinone** (5)

Selon les conditions de l'exemple 1, on agite 5 g (28,7 mmoles) de juglone et 5,54 g (28,7 mmoles) de 2-phénylindole pendant 4 heures pour obtenir 4,72 g (12,9 mmoles) de 3-(2-phényl 3-indolyl)5-hydroxy 1,4-naphtoquinone (5). PF. 120°C (déc.). Coloration violette.

### EXEMPLE 6

**2-(2-phényl 3-indolyl)3-phényl 1,4-naphtoquinone** (6)

Les conditions de l'exemple 1 appliquées à 2 g (8,55 mmoles) de 2-phényl 1,4-naphtoquinone et 1,10 g (5,70 mmoles) de 2-phényl indole conduisent, après 240 heures, à 0,51 g (1,20 mmole) de 2-(2-phényl 3-indolyl)3-phényl 1,4-naphtoquinone (6). PF. 131 °C. Coloration bleue.

EXEMPLE 7

**2-(3-indolyl)5,8-dihydroxy 1,4-naphtoquinone** (7)

Selon les conditions de l'exemple 1, 2 g (10,52 mmoles) de 5,8-dihydroxy 1,4-naphtoquinone réagissent avec 3,70 g (31,55 mmoles) d'indole pendant 1440 heures. On isole, par chromatographie sur plaque des cristaux rouges de 2-(3-indolyl)5,8-dihydroxy 1,4-naphtoquinone (7) ayant un point de fusion de 230°C.

EXEMPLE 8

**2-(1-méthyl 3-indolyl)3-phényl 1,4-naphtoquinone** (8)

Pour obtenir cette indolylnaphtoquinone, on opère selon les conditions de l'exemple 1, mais on porte la température à 70°C par un bain d'huile thermostaté. Dans ces conditions, 2 g (8,55 mmoles) de 2-phényl 1,4-naphtoquinone réagissent avec 1,65 ml (12,9 mmoles) de 1-méthylindole pour conduire, après avoir traité et purifié le mélange, à 0,20 g (0,55 mmoles) de 2-(1-méthyl 3-indolyl)3-phényl 1,4-naphtoquinone (8). PF. 180°-183°C. Coloration bleue.

EXEMPLE 9

**2-(2-méthyl 3-indolyl)3-(1-pentyl)1,4-naphtoquinone** (9)

En opérant avec de l'acide acétique glacial à 70°C, 5 g (21,93 mmoles) de 2-(1-pentyl)1,4-naphtoquinone réagissent pendant 120 heures sur 4,31 g (32,89 mmoles) de 2-méthylindole pour former 1,02 g (2,88 mmoles) de 2-(2-méthyl 3-indolyl)3-(1-pentyl)1,4-naphtoquinone (9) se présentant sous la forme d'une pâte brune. Coloration bleue.

EXEMPLE 10

**3-(1-undécyl)2-(1,2-diméthyl 3-indolyl)1,4-naphtoquinone** (10)

Selon les conditions de l'exemple 9, 3 g (9,6 mmoles) de 2-(1-undécyl) 1,4-naphtoquinone réagissent avec 1,40 g (9,6 mmoles) de 1,2-diméthylindole pour conduire, après 72 heures, à 2 g (4,4 mmoles) de l'indolylquinone (10) sous forme d'une pâte brune. Coloration bleue.

EXEMPLE 11

**3-(1-undécyl)2-(2-phényl 3-indolyl)1,4-naphtoquinone** (11)

Selon les conditions de l'exemple 9, 3 g (9,6 mmoles) de 2-(1-undécyl) 1,4-naphtoquinone et 1,85 g (9,6 mmoles) de 2-phénylindole fournissent après 72 heures, 0,50 g (1 mmole) de 3-(1-undécyl) 2-(2-phényl 3-indolyl)1,4-naphtoquinone (11) se présentant sous la forme d'une pâte brune amorphe. Coloration bleue.

EXEMPLE 12

**2-(2-méthyl 3-indolyl)3-(1-pipéridyl)1,4-naphtoquinone** (12)

Dans les conditions de l'exemple 9, 1,50 g (6,22 mmoles) de 2-(1-pipéridyl) 1,4-naphtoquinone et 2,60 g (19,8 mmoles) de 2-méthyl indole fournissent, au bout de 168 heures, des cristaux rouges de 2-(2-méthyl 3-indolyl)3-(1-pipéridyl)1,4-naphthoquinone. PF. 135°C. Coloration rouge.

EXEMPLE 13

**2-(1,2-diméthyl 3-indolyl)3-(1-pipéridyl)1,4-naphtoquinone** (13)

En procédant comme à l'exemple 9, 1,50 g (6,22 mmoles) de 2-(1-pipéridyl)1,4-naphtoquinone et 1,36 g (9,37 mmoles) de 1,2-diméthylindole fournissent, après 144 heures de chauffage et d'agitation, 100 mg (0,26 mmole) de 2-(1,2-diméthyl 3-indolyl) 3-(1-pipéridyl) 1,4-naphtoquinone (13). PF. 164°C. Coloration rouge.

EXEMPLE 14

**2-(1-méthyl 3-indolyl)3-thiophényl 1,4-naphtoquinone** (14)

En suivant le mode opératoire de l'exemple 9, 1,50 g (5,64 mmoles) de 2-thiophényl 1,4-naphtoquinone donnent, avec 1,08 ml (8,45 mmoles) de 1-méthylindole pendant 24 heures, 220 mg (0,55 mmole) de 2-(1-méthyl 3-indolyl) 3-thiophényl 1,4-naphtoquinone (14). PF. 183°-185°C. Coloration violette.

EXEMPLE 15

**2-(2-méthyl 3-indolyl)3-thiophényl 1,4-naphtoquinone** (15)

Dans les conditions de l'exemple 9, 1,50 g (5,04 mmoles) de 2-thiophényl 1,4-naphtoquinone réagissent avec 1,11 g (8,46 mmoles) de 2-méthylindole pendant 240 heures pour donner 313 mg (0,79 mmole) de 2-(2-méthyl 3-indolyl)3-thiophényl 1,4-naphtoquinone (15). PF. 173°C. Coloration violette.

EXEMPLE 16

**Synthèse de la 2-(2-phényl 3-indolyl)3-méthoxy 1,4-naphtoquinone** (16)

Dans un ballon tricol de 100 ml purgé à l'argon et surmonté d'un réfrigérant, on agite fortement 1,50 g (7,98 mmoles) de 2-méthoxy 1,4-naphtoquinone avec 4,63 g (23,93 mmoles), de 2-phénylindole dans 25 ml de chloroforme porté au reflux par un bain d'huile thermostaté. Le déroulement de la réaction est suivi par chromatographie analytique sur plaque de gel de silice. Après 72 heures d'agitation, on ajoute 50 ml de chloroforme supplémentaires, sèche sur sulfate de magnésium calciné sec, et évapore le solvant sous vide. Le résidu noir obtenu est chromatographié sur colonne de gel de silice éluée au dichlorométhane pour conduire à 0,15 g (0,40 mmole) de 2-(2-phényl 3-indolyl)3-méthoxy 1,4-naphtoquinone (16). PF. 88°-90°C. Coloration rouge.

EXEMPLE 17

**Synthèse de la 3-(1-méthyl 3-indolyl)5-hydroxy 1,4-naphtoquinone** (17)

Dans un ballon tricol de 250 ml, on dissout dans 50 ml de chloroforme 5 g (28,74 mmoles) de 5-hydroxy 1,4-naphtoquinone (juglone) purifiée sur colonne et 3,67 ml (28,7 mmoles) de 1-méthylindole en présence de 0,50 g (2,6 mmoles) d'acide paratoluène sulfonique monohydraté. On laisse agiter 48 heures pendant lesquelles on suit la réaction par chromatographie analytique sur plaque de gel de silice. Puis on ajoute 100 ml de chloroforme et 50 ml d'eau. On décante, neutralise la phase organique, sèche sur sulfate de magnésium, évapore le solvant sous vide et chromatographie le résidu noir obtenu sur colonne de gel de silice pour donner 1,83 g (6,04 mmoles) de 3-(1-méthyl 3-indolyl)5-hydroxy 1,4-naphtoquinone (17). PF. 224°C. Coloration violette.

EXEMPLE 18

**Préparation de la 2-(2-méthyl 3-indolyl)3-phényl 1,4-naphtoquinone** (18)

On opère comme pour l'exemple 17, sauf pour la température de la réaction qui est portée au reflux du chloroforme. Ainsi, 3 g (12,82 mmoles) de 2-phényl 1,4-naphtoquinone et 2,52 g (19,21 mmoles) de 2-méthylindole sont chauffés pendant 3 heures en présence de 0,375 g (1,95 mmole) d'acide paratoluène sulfonique monohydraté pour conduire à 1 g (2,75 mmoles) de 2-(2-méthyl 3-indolyl)3-phényl 1,4-naphtoquinone (18). PF.115°-121°C. Coloration bleue.

EXEMPLE 19

**2-(2-méthyl 3-indolyl)3-(1-adamantyl)méthyl 1,4-naphtoquinone** (19)

En opérant comme dans l'exemple 18, 0,50 g (1,63 mmole) de 2-(1-adamantyl)méthyl 1,4-naphtoquinone réagissent avec 0,22 g (1,63 mmole) de 2-méthylindole pour fournir 0,126 g (0,29 mmole) de l'indolylnaphtoquinone (19). PF. 136°C. Coloration bleue.

EXEMPLE 20

**2-(1-méthyl 3-indolyl)3-chloro 1,4-naphtoquinone** (20)

En suivant les conditions de l'exemple 18,1 g (5,2 mmoles) de la 2-chloro 1,4-naphtoquinone réagit avec 1,2 ml (9,4 mmoles) de 1-méthylindole en présence d'acide paratoluène sulfonique monohydrate pour donner deux produits principaux : 36 mg (0,12 mmole) de 2-(1-méthyl 3-indolyl) 1,4-naphtoquinone, accompagné de 80 mg (0,25 mmole) de 2-(1-méthyl 3-indolyl)3-chloro 1,4-naphtoquinone. PF. 205°C.

EXEMPLE 21

**Synthèse de la 2-(2-méthyl 3-indolyl)3-hydroxy 1,4-naphtoquinone** (21)

Dans un tricol d'1 litre surmonté d'un réfrigérant, d'un thermomètre et d'une arrivée d'argon, sont placés 25 g (144 mmoles) de Lawsone et 28,2 g (216 mmoles) de 2-méthylindole dans 400 ml d'éthanol absolu. Le mélange est porté au reflux du solvant et agité pendant 96 heures. Puis on évapore le solvant et chromatographie le résidu noir sur colonne de gel de silice éluée au dichlorométhane. On obtient 1,6 g (5,3 mmoles) de 2-(2-méthyl 3-indolyl)3-hydroxy 1,4-naphtoquinone (21). PF. > 330°C. Coloration rouge.

EXEMPLE 22

**2-bromo 3-(2-méthyl 3-indolyl)1,4-naphtoquinone** (22)

a) Dans un ballon purgé à l'argon, on agite 13,30 g (50 mmoles) de 2-bromo 3-méthoxy 1,4-naphtoquinone et 6,56 g (50 mmoles) de 2-méthylindole dans 250 ml d'éthanol absolu. On porte au reflux pendant 72 heures, puis évapore le solvant. Le résidu obtenu est chromatographié sur colonne de gel de silice. On obtient ainsi 14,27 g (40 mmoles) de 2-bromo 3-(2-méthyl 3-indolyl) 1,4-naphtoquinone. PF. 113°-115°C.
b) En procédant comme ci-dessus, 15,80 g (50 mmoles) de 2,3-dibromo 1,4-naphtoquinone réagissent sur 6,56 g (50 mmoles) de 2-méthylindole pour fournir 11,34 g (31 mmoles) de 2-bromo 3-(2-méthyl 3-indolyl) 1,4-naphtoquinone (22). PF. 112°-114°C. Coloration violette.

EXEMPLE 23

**2-(2-méthyl 3-indolyl)1,4-dihydroxynaphtalène** (23)

A une solution de 1 g (3,48 mmoles) de 2-(2-méthyl 3-indolyl) 1,4-naphtoquinone dans 50 ml d'éther éthylique placée sous argon, on ajoute 1,68 g (2 éq.) de dithionite de sodium en solution dans 15 ml d'eau. Le mélange est agité sous argon à température ambiante pendant 12 heures. La solution organique incolore est extraite et séchée sur sulfate de magnésium et le solvant évaporé. On obtient 1,01 g (3,48 mmoles) de 2-(2-méthyl 3-indolyl)1,4-dihydroxynaphtalène (23).

Dans les tableaux 1 et 2 ci-après figurent, respectivement, les caractéristiques spectrales IR et UV-visible des composés synthétisés ci-dessus.

**TABLEAU 1**

| Valeurs des fréquences d'élongation γ C=O et γ N-H ($cm^{-1}$) des indolyl naphtoquinones dans le chloroforme. 99,8%. | | |
|---|---|---|
| Composés | γC=O | γN-H |
| (1) | 1675,1650 | - |
| (2) | 1671,1650 | 3456 |
| (3) | 1720,1665,1648 | - |
| (17) | 1640 | - |
| (4) | 1640 | 3470 |
| (5) | 1638 | 3452 |
| (18) | 1670 | 3475 |
| (6) | 1670 | 3470 |
| (8) | 1665 | - |
| (9) | 1660 | 3480 |
| (10) | 1665 | - |
| (11) | 1665 | 3470 |
| (19) | 1655 | 3470 |
| (21) | 1650,1640 | 3390 |
| (16) | 1650 | 3460 |
| (20) | 1670 | - |
| (22) | 1680 | 3470 |
| (12) | 1665 | 3460 |
| (13) | 1665 | - |
| (14) | 1660 | - |
| (15) | 1660 | 3460 |

## TABLEAU 2

Absorption UV-visible des indolylnaphtoquinones.
(ε = coefficient d'extinction molaire).

| Composés | λ max (nm) ($\log_{10}$ ε) | Solution * |
|---|---|---|
| (1) | 224(4,57);279(4,62);287(4,63);502(4,12) | 1 |
| (2) | 211(4,46);241(4,40);287(4,25);514(3,42) | 1 |
| (3) | 246(4,28;272(4,25);281(4,26);290(4,24) 342(3,50);525(3,58) | 1 |
| (17) | 212(4,73);288(4,40);428(3,88);527(3,93) | 1 |
| (4) | 212(4,63);282(4,20);426(3,62);553(3,50) | 1 |
| (5) | 201(4,20);212(4,53);240(4,36);285(4,25) 417(3,64);525(3,38) | 3 |
| (18) | 222(4,67);246(4,54);286(4,33);294(4,33) 556(3,40) | 1 |
| (6) | 210(4,49);243(4,43);304(4,27);528(2,86) | 3 |
| (8) | 204(4,57);218(4,45);293(4,09);529(3,33) | 1 |
| (9) | 202(4,53);221(4,61);249(4,30);270(4,31) 516(3,12) | 1 |
| (10) | 208(4,31);225(4,39);272(3,96);513(2,76) | 2 |
| (11) | 211(4,32);241(4,29);275(4,06);303(4,10) 513(2,78) | 1 |
| (19) | 223(4,45);278(4,10);288(4,05);517(2,95) | 2 |
| (21) | 206(4,87);224(4,90);275(4,73);542(3,50) | 1 |
| (16) | 204(4,60);220(4,56);241(4,50);284(4,34) 512(3,16) | 2 |
| (20) | 210(4,51);219(4,52);246(4,19);251(4,20) 278(4,22);287(4,23); 513(3,59) | 3 |
| (22) | 202(4,56);219(4,61);246(4,18);252(4,16) 279(4,28);558(3,33) | 2 |
| (12) | 226(4,51);283(4,28);290(4,28);485(3,46) 527(3,46) | 2 |

| | | |
|---|---|---|
| (13) | 211(4,30);230(4,39);294(4,11);485(3,23) 535(3,58) | 2 |
| (14) | 209(4,44);252(4,38);273(4,38);290(4,38) 433(4,36);525(3,51) | 2 |
| (15) | 210(4,44);216(4,49);264(4,20);432(3,33) 558(3,58) | 2 |

| | | |
|---|---|---|
| * Solution | 1 | Ethanol à 99,8% |
| | 2 | Ethanol à 99,5% |
| | 3 | Tétrahydrofurane à 99,7% |

**EXEMPLES DE COMPOSITIONS**

EXEMPLE A

**Composition tinctoriale pour cheveux**

- 2-(2-méthyl 3-indolyl)5-hydroxy 1,4-naphtoquinone 0,1 g
- Copolymère acétate de vinyle/acide crotonique/polyéthylèneglycol, vendu sous la dénomination ARISTOFLEX A par la Société HOECHST 1,5 g
- Alcool éthylique absolu 40 g
- Triéthanolamine qsp pH=7
- Eau déminéralisée qsp 100 g

On applique soigneusement cette lotion de mise en plis sur des cheveux à 100% blancs. Les cheveux sont alors mis en forme et séchés. Ils sont colorés en gris clair très nacré.

**Revendications**

**1.** Composition de teinture des fibres kératiniques, en particulier des cheveux humains, caractérisée par le fait qu'elle contient dans un milieu approprié pour la teinture, au moins un composé répondant à l'une des formules suivantes :

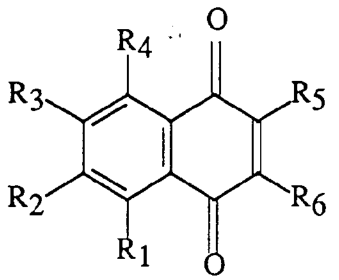

ou

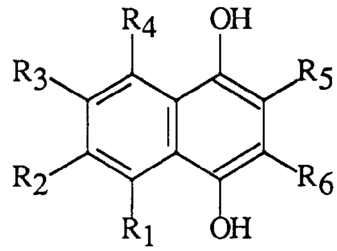

(I) (II)

dans lesquelles :

$R_1$ à $R_4$, identiques ou différents, désignent un atome d'hydrogène, un groupe OH, un radical alcoxy en $C_1$-$C_4$; deux au plus pouvant désigner OH;

$R_5$ ou $R_6$ désignent un atome d'hydrogène, OH, un halogène, un radical alkyle en $C_1$-$C_{12}$, alcoxy en $C_1$-$C_4$, phényle, thiophényle, adamantylméthyle, adamantylamino, alkyl($C_1$-$C_4$)amino, pipéridyle, morpholinyle ou un radical R de formule :

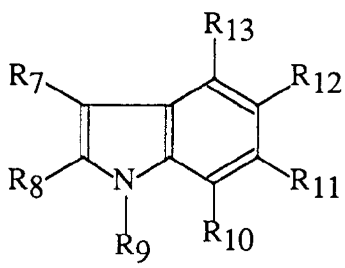

(III)

où

$R_7$ ou $R_8$ désignent un atome d'hydrogène, un radical alkyle en $C_1$-$C_4$, phényle ou une liaison covalente; un des symboles $R_7$ et $R_8$ désigne une liaison covalente;

$R_9$ désigne un atome d'hydrogène ou un radical alkyle en $C_1$-$C_4$;

$R_{10}$ à $R_{13}$, identiques ou différents, désignent un atome d'hydrogène, un radical alkyle en $C_1$-$C_4$, un halogène, un radical alcoxy en $C_1$-$C_4$, alcoxy($C_1$-$C_4$)carbonyle, acyle en $C_2$-$C_4$, acyl ($C_2$-$C_4$)oxy, $NO_2$, OH, amino éventuellement monosubstitué par un alkyle en $C_1$-$C_4$ ou hydroxyalkyle en $C_1$-$C_4$, cyano ou benzyloxy;

au plus un seul des radicaux $R_{10}$ à $R_{13}$ peut désigner halogène, $NO_2$, alcoxycarbonyle, acyle, cyano, amino ou acyloxy;

au plus deux des radicaux $R_{10}$ à $R_{13}$ peuvent désigner OH, alcoxy ou benzyloxy;

$R_{11}$ et $R_{12}$ peuvent former un cycle méthylènedioxy;

un des radicaux $R_8$ et $R_6$ désigne un radical R.

2. Composition selon la revendication 1, caractérisée par le fait que les composés de formule (I) ou (II) sont choisis parmi ceux pour lesquels :

$R_1$ et $R_4$ désignent hydrogène, OH ou alcoxy;

$R_2$ et $R_3$ désignent hydrogène ou OH;

deux au plus des symboles $R_1$ à $R_4$ pouvant désigner OH;

$R_5$ ou $R_8$ désignent hydrogène, phényle, alkyle en $C_1$-$C_{12}$, adamantylméthyle, OH, méthoxy, chlore, brome, pipéridyle, thiophényle ou le radical R de formule :

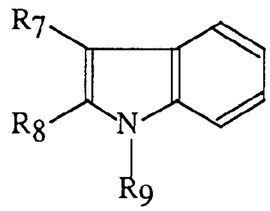

(III')

où

$R_9$ désigne hydrogène ou méthyle;
$R_7$ ou $R_8$ désignent hydrogène, méthyle, phényle ou une liaison covalente;
l'un des symboles $R_7$ et $R_8$ désigne une liaison covalente et l'un des radicaux $R_5$ et $R_6$ désigne R.

**3.** Composition selon la revendication 1 ou 2, caractérisée par le fait qu'elle contient au moins un composé choisi parmi :

- la 2-(1-méthyl 3-indolyl)1,4-naphtoquinone,
- la 2-(2-méthyl 3-indolyl)1,4-naphtoquinone,
- la 2-(2-phényl 3 -indolyl)1,4-naphtoquinone,
- la 2-(1,2-diméthyl 3-indolyl)1,4-naphtoquinone,
- la 3-(1-méthyl 3-indolyl)5-hydroxy 1,4-naphtoquinone,
- la 2-(2-méthyl 3-indolyl)5-hydroxy 1,4-naphtoquinone,
- la 3-(2-phényl 3-indolyl)5-hydroxy 1,4-naphtoquinone,
- la 2-(2-méthyl 3-indolyl)3-phényl 1,4-naphtoquinone,
- la 2-(2-phényl 3-indolyl)3-phényl 1,4-naphtoquinone,
- la 2-(1-méthyl 3-indolyl)3-phényl 1,4-naphtoquinone,
- la 2-(2-méthyl 3-indolyl)3-(1-pentyl)1,4-naphtoquinone,
- la 3-undécyl 2-(1,2-diméthyl 3-indolyl)1,4-naphtoquinone,
- la 3-undécyl 2-(2-phényl 3-indolyl)1,4-naphtoquinone,
- la 2-(2-méthyl 3-indolyl)3-(1-adamantyl)méthyl 1,4-naphtoquinone,
- la 2-(2-méthyl 3-indolyl)3-hydroxy 1,4-naphtoquinone,
- la 2-(2-phényl 3-indolyl)3-méthoxy 1,4-naphtoquinone,
- la 2-(1-méthyl 3-indolyl)3-chloro 1,4-naphtoquinone,
- la 3-(2-méthyl 3-indolyl)2-bromo 1,4-naphtoquinone,
- la 2-(2-méthyl 3-indolyl)3-(1-pipéridyl)1,4-naphtoquinone,
- la 2-(1,2-diméthyl 3-indolyl)3-(1-pipéridyl)1,4-naphtoquinone,
- la 2-(1-méthyl 3-indolyl)3-thiophényl 1,4-naphtoquinone,
- la 2-(2-méthyl 3-indolyl)3-thiophényl 1,4-naphtoquinone,
- la 2-(3-indolyl)5,8-dihydroxy 1,4-naphtoquinone,
- la 2-(3-méthyl 2-indolyl)1,4-naphtoquinone,

ainsi que leurs dérivés de réduction correspondants de structure 1,4-dihydroxynaphtalène.

**4.** Composition selon l'une quelconque des revendications 1 à 3, caractérisée par le fait que les composés de formule (I) ou (II) sont présents à des concentrations comprises entre 0,01 et 10% en poids par rapport au poids total de la composition.

**5.** Composition selon l'une quelconque des revendications 1 à 4, caractérisée par le fait que le milieu approprié pour la teinture est constitué par de l'eau, un solvant ou un mélange d'eau et d'un ou plusieurs solvant(s) choisi(s) parmi les alcools inférieurs en $C_1$-$C_6$, les glycols comme l'éthylèneglycol, le propylèneglycol, les éthers de glycol comme les éthers monométhylique, monoéthylique ou monobutylique de l'éthylèneglycol, l'acétate du monoéthyléther de l'éthylèneglycol, les monométhyléthers du propylèneglycol et du dipropylèneglycol, le lactate de méthyle.

**6.** Composition selon l'une quelconque des revendications 1 à 5, caractérisée par le fait qu'elle contient en plus des colorants directs autres que ceux de formules (I) ou (II), des agents tensio-actifs anioniques, cationiques, non-ioniques, amphotères ou leurs mélanges, des épaississants, des parfums, des séquestrants, des agents filmogènes, des agents de traitement des fibres kératiniques, des agents dispersants, des agents de conditionnement des fibres kératiniques, des conservateurs, des agents de gonflement des fibres kératiniques.

7. Composition selon l'une quelconque des revendications 1 à 6, caractérisée par le fait qu'elle se présente sous forme de lotion plus ou moins épaissie ou gélifiée, de dispersion, de crème, de mousse, ou qu'elle est conditionnée dans un dispositif aérosol.

8. Composé répondant à l'une des formules suivantes :

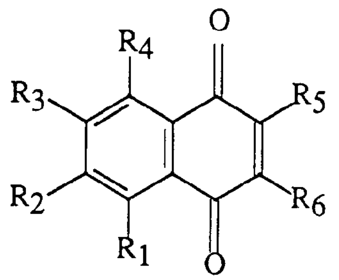

ou

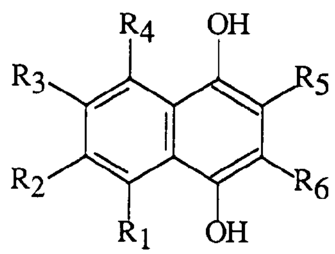

(I) (II)

dans lesquelles :

$R_1$ à $R_4$, identiques ou différents, désignent un atome d'hydrogène, un groupe OH, un radical alcoxy en $C_1$-$C_4$; deux au plus pouvant désigner OH;

$R_5$ ou $R_6$ désignent un atome d'hydrogène, OH, un halogène, un radical alkyle en $C_1$-$C_{12}$, alcoxy en $C_1$-$C_4$, phényle, thiophényle, adamantylméthyle, adamantylamino, alkyl($C_1$-$C_4$)amino, pipéridyle, morpholinyle ou un radical R de formule :

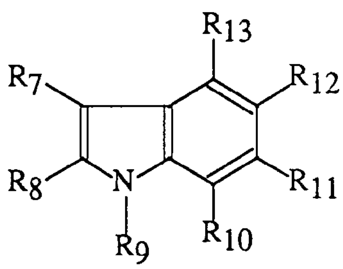

(III)

où

$R_7$ ou $R_8$ désigne un atome d'hydrogène, un radical alkyle en $C_1$-$C_4$, phényle ou une liaison covalente; un des symboles $R_7$ et $R_8$ désigne une liaison covalente;

$R_9$ désigne un atome d'hydrogène ou un radical alkyle en $C_1$-$C_4$;

$R_{10}$ à $R_{13}$, identiques ou différents, désignent un atome d'hydrogène, un radical alkyle en $C_1$-$C_4$, un halogène, un radical alcoxy en $C_1$-$C_4$, alcoxy($C_1$-$C_4$)carbonyle, acyle en $C_2$-$C_4$, acyl ($C_2$-$C_4$)oxy, $NO_2$, OH, amino éventuellement monosubstitué par un alkyle en $C_1$-$C_4$ ou un hydroxyalkyle en $C_1$-$C_4$, cyano ou benzyloxy;

au plus un seul des radicaux $R_{10}$ à $R_{13}$ peut désigner halogène, $NO_2$, alcoxycarbonyle, acyle, cyano, amino ou acyloxy;

au plus deux des radicaux $R_{10}$ à $R_{13}$ peuvent désigner OH, alcoxy ou benzyloxy;

$R_{11}$ et $R_{12}$ peuvent former un cycle méthylènedioxy;

un des radicaux $R_8$ et $R_6$ désigne un radical R;

sous réserve que :

a) $R_{11}$ et $R_{12}$ ne peuvent désigner simultanément OH lorsque $R_1$ à $R_4$, $R_6$, $R_{10}$ et $R_{13}$ désignent hydrogène et, soit $R_8$ et $R_9$ désignent hydrogène, soit l'un des radicaux $R_8$ et $R_9$ désigne $CH_3$ et l'autre désigne H;

b) $R_8$ ne peut désigner méthyle, lorsque $R_1$ à $R_4$, $R_6$, $R_9$ et $R_{10}$ a $R_{13}$ désignent hydrogène et $R_7$ désigne une liaison covalente;

c) $R_7$ ne peut désigner méthyle lorsque $R_1$ à $R_4$, $R_6$, $R_9$ et $R_{10}$ a $R_{13}$ désignent hydrogène et $R_8$ désigne une liaison covalente;

d) au moins l'un des radicaux $R_1$ à $R_4$, $R_8$ ou $R_8$ et $R_8$ à $R_{13}$ est différent d'hydrogène lorsque $R_7$ désigne

une liaison covalente;
e) $R_9$ ne peut désigner méthyle lorsque $R_5$ désigne méthoxy et $R_1$ à $R_4$, $R_7$ ou $R_8$ et $R_{10}$ à $R_{13}$ désignent hydrogène.

**9.** Procédé de teinture directe des fibres kératiniques, en particulier des cheveux humains, caractérisé par le fait qu'on applique sur les fibres au moins une composition telle que définie selon l'une quelconque des revendications 1 à 8, l'application étant éventuellement suivie d'un rinçage.

**10.** Procédé de préparation des composés de formule (I) définie dans la revendication 8, caractérisé par le fait que l'on fait réagir un composé indolique de formule (1) :

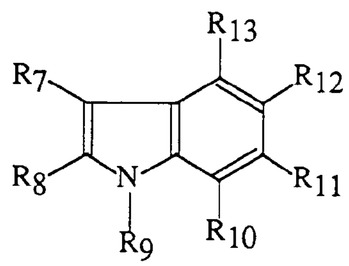

(1)

dans laquelle :
$R_7$ ou $R_8$ désigne un atome d'hydrogène, un radical alkyle en $C_1$-$C_4$, phényle;
l'un de ces radicaux désignant l'hydrogène;
$R_9$ à $R_{13}$ ont les mêmes significations indiquées dans la formule (III) de la revendication 8 avec une 1,4-naphtoquinone de formule (2) :

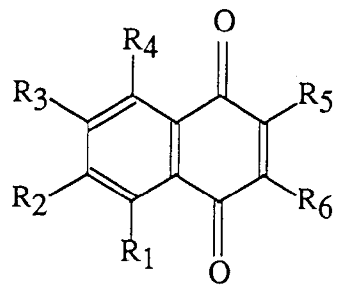

(2)

dans laquelle :
$R_1$ à $R_4$ ont les mêmes significations indiquées dans la formule (I) de la revendication 8;
$R_5$ ou $R_8$ désigne un atome d'hydrogène, OH, halogène, un alkyle en $C_1$-$C_{12}$, alcoxy en $C_1$-$C_4$, phényle, thiophényle, adamantylméthyle, adamantylamino, alkylamino en $C_1$-$C_4$, pipéridyle, morpholinyle; l'un au moins des radicaux $R_5$ et $R_8$ désignant l'hydrogène,
dans un milieu constitué par de l'acide acétique glacial, de l'acide acétique aqueux, de l'éthanol ou du chloroforme contenant éventuellement de l'acide p-toluène sulfonique à une température variant de la température ambiante à celle du reflux du mélange.

**11.** Procédé de préparation des composés de formule (I) définie dans la revendication 8, caractérisé par le fait que l'on fait réagir un composé indolique de formule (1) :

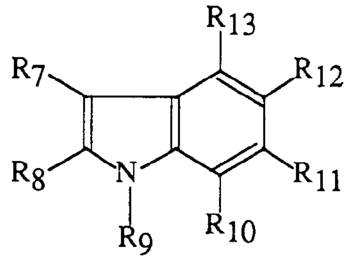

(1)

dans laquelle :

$R_7$ ou $R_8$ désigne un atome d'hydrogène, un radical alkyle en $C_1$-$C_4$, phényle;

l'un de ces radicaux désignant l'hydrogène;

$R_9$ à $R_{13}$ ont les mêmes significations indiquées dans la formule (III) de la revendication 8 avec une 1,4-naphtoquinone de formule (2) :

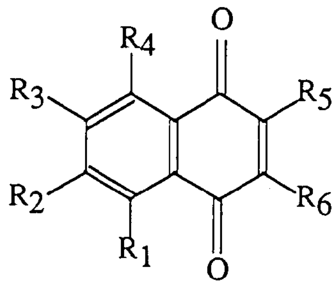

(2)

dans laquelle :

$R_1$ à $R_4$ ont les mêmes significations indiquées dans la formule (I) de la revendication 8;

$R_5$ ou $R_6$ désignent OH, halogène, un alkyle en $C_1$-$C_{12}$, alcoxy en $C_1$-$C_4$, phényle, thiophényle, adamantylméthyle, adamantylamino, alkylamino en $C_1$-$C_4$, pipéridyle, morpholinyle;

au moins un des radicaux $R_6$ et $R_6$ désigne OH, Cl, Br, méthoxy, thiophényle ou pipéridyle;

dans de l'éthanol au reflux.

Office européen des brevets

# RAPPORT DE RECHERCHE EUROPEENNE

Numero de la demande

EP 93 40 0625

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,A | EP-A-0 460 996 (L'ORÉAL)<br>* revendications *<br>--- | 1 | C07D209/12<br>A61K7/13<br>C07D409/10 |
| D,A | EP-A-0 376 776 (L'ORÉAL)<br>* revendications *<br>----- | 1 | |
| | | | **DOMAINES TECHNIQUES RECHERCHES (Int. Cl.5 )** |
| | | | C07D<br>A61K |

Le présent rapport a été établi pour toutes les revendications

| Lieu de la recherche | Date d'achèvement de la recherche | Examinateur |
|---|---|---|
| LA HAYE | 17 JUIN 1993 | VAN BIJLEN H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)